Europäisches Patentamt

European Patent Office

Office européen des brevets

(11) Veröffentlichungsnummer : **0 405 348 B1**

(12) # EUROPÄISCHE PATENTSCHRIFT

(45) Veröffentlichungstag der Patentschrift :
**28.07.93 Patentblatt 93/30**

(51) Int. Cl.$^5$ : **C07C 47/052,** B01J 23/04

(21) Anmeldenummer : **90111840.6**

(22) Anmeldetag : **22.06.90**

(54) **Verfahren zur Herstellung von Formaldehyd.**

(30) Priorität : **24.06.89 DE 3920811**

(43) Veröffentlichungstag der Anmeldung :
**02.01.91 Patentblatt 91/01**

(45) Bekanntmachung des Hinweises auf die
Patenterteilung :
**28.07.93 Patentblatt 93/30**

(84) Benannte Vertragsstaaten :
**BE DE ES FR GB IT NL**

(56) Entgegenhaltungen :
**EP-A- 0 294 684**
**US-A- 2 249 380**
**CHEMICAL ABSTRACTS, Band 113, Nr. 2, 9.**
**Juli 1990, Seite 113, Zusammenfassung Nr.**
**8371u, Columbus, Ohio, US; G. WIESGICKL et**
**al: "Novel type of catalyst for the pure dehy-**
**drogenation of methanol to formaldehyde"**

(56) Entgegenhaltungen :
**CHEMICAL ABSTRACTS, Band 106, Nr. 23, 8.**
**June 1987, Seite 689, Zusammenfassung Nr.**
**195844r, Columbus, Ohio, US; Y. MATSUMURA**
**et al: "Dehydrogenation of methanol to for-**
**maldehyde over silicalite"**
**PATENT ABSTRACTS OF JAPAN, Band 12, Nr.**
**299 (C-520)(3146), 15. August 1988; & JP-**
**A-6369542**

(73) Patentinhaber : **HOECHST**
**AKTIENGESELLSCHAFT**
**Postfach 80 03 20**
**W-6230 Frankfurt am Main 80 (DE)**

(72) Erfinder : **Beck, Horst-Philipp, Prof. Dr.**
**Scheidter Strasse 158**
**W-6600 Saarbrücken (DE)**
Erfinder : **Emig, Gerhard, Prof. Dr.**
**Vogelherd 157**
**W-8520 Erlangen (DE)**
Erfinder : **Wiesgickl, Günther, Dr.**
**Schillerring 23**
**W-8751 Grosswallstadt (DE)**
Erfinder : **Burg, Karlheinz, Dr.**
**Eichenweg 18**
**W-6200 Wiesbaden (DE)**
Erfinder : **Mück, Karl-Friedrich, Dr.**
**Schnitterweg 7**
**W-6200 Wiesbaden (DE)**

EP 0 405 348 B1

## Beschreibung

Die vorliegende Erfindung betrifft ein Verfahren zur Herstellung von Formaldehyd durch Dehydrierung von Methanol in Gegenwart eines Katalysators bei erhöhter Temperatur.

Es sind mehrere Verfahren zur Herstellung von Formaldehyd aus Methanol bekannt. In der Technik ist die Oxydation von Methanol zu Formaldehyd nach folgender Formel üblich

$$CH_3OH + 1/2\ O_2 \rightarrow CH_2O + H_2O \quad (I)$$

die an Eisen- und Molybdänoxyd enthaltenden Katalysatoren bei 350°C bis 450°C durchgeführt wird. Ebenfalls üblich ist die oxydative Dehydrierung von Methanol zu Formaldehyd gemäß den Gleichungen

$$CH_3OH \rightleftarrows CH_2O + H_2 \quad (IIa)$$
$$H_2 + 1/2\ O_2 \rightleftarrows H_2O \quad (IIb)$$

an Silberkatalysatoren bei 600 bis 720°C. Beide Verfahren werden beschrieben in Ullmanns Encycl. der techn. Chemie, Band 11, S. 693-694, 4. Auflage, 1976, Verlag Chemie Weinheim.

So ist beispielsweise ein Verfahren zur Herstellung von Formaldehyd aus Methanol durch Dehydrierung bei erhöhter Temperatur bekannt, bei dem die Umsetzung in Gegenwart eines mindestens eine Natriumverbindung enthaltenden Katalysators bei einer Temperatur von 300°C bis 800°C durchgeführt wird (DE-A 37 19 055).

In einer anderen Veröffentlichung, in der die Verwendung eines Silberoxid enthaltenden Katalysators erwähnt wird, ist ausgeführt, daß auf die Abwesenheit von $Al_2O_3$ und MgO als Verunreinigung geachtet werden muß (JP-A 60 089-441 = Derwent Report 85-156891/26).

Die bekannten Katalysatoren ermöglichen jedoch keine wirtschaftliche Herstellung von Formaldehyd durch Dehydrierung von Methanol. Bei den bekannten Verfahren betragen die Ausbeuten an Formaldehyd in allen Fällen weniger als 70 % und die Umsätze, d.h. das Verhältnis von umgesetztem Methanol zu zudosiertem Methanol, waren niemals größer als 95 %. Es war daher wünschenswert,gleich gute oder bessere Ergebnisse mit billigeren und einfach herzustellenden Katalysatoren zu erzielen.

Die Erfindung betrifft daher ein Verfahren zur Herstellung von Formaldehyd durch Dehydrierung von Methanol in Gegenwart eines Katalysators bei erhöhter Temperatur, wobei die Umsetzung bei einer Temperatur von 650 bis 1050°C unter Sauerstoffausschluß in Gegenwart von Aluminiumoxid, Alkalialuminat und/oder Erdalkalialuminat als Katalysator in einem Reaktor, dessen Innenwand ganz oder teilweise aus Aluminiumoxid besteht, durchgeführt wird.

Die Dehydrierungs-Reaktion kann durch die Gleichung

$$CH_3OH \rightleftarrows CH_2O + H_2 \quad (IIa)$$

beschrieben werden.

Das Verfahren liefert überraschenderweise Formaldehyd mit guter Selektivität und Ausbeute.

Zur Herstellung des Katalysators eignen sich Aluminiumverbindungen wie etwa die Oxyde, Hydroxyde, Carbonate, Bicarbonate, Oxalate, Acetate oder Nitrate allein oder in Mischung mit entsprechenden Alkali- oder Erdalkaliverbindungen.

Als Kationen der Alkali- und Erdalkaliverbindungen kommen solche der 1. und 2. Hauptgruppe des Periodischen Systems in Frage wie Lithium, Natrium, Kalium, Rubidium, Magnesium, Calcium, Strontium und Barium, vorzugsweise aber Lithium und Natrium oder Mischungen daraus. Der Katalysator kann in einer technisch üblichen Form verwendet werden, beispielsweise in Form von Kugeln oder Stäbchen. Er kann unmittelbar, d.h. in der Ausgangsform der Verbindungen eingesetzt werden oder zuvor thermisch, beispielsweise an der Luft und/oder chemisch aktiviert werden. Auf jeden Fall werden die Ausgangsverbindungen bei den hohen Temperaturen im Reaktor in die aktive Form übergeführt. Anionische Gruppen wie Carbonat, Bicarbonat, Oxalat, Acetat oder Nitrat werden nach der Behandlung bei Temperaturen, die den Reaktionstemperaturen entsprechen, nicht mehr nachgewiesen.

Die Reaktionstemperaturen bei der Dehydrierung des Methanols betragen im allgemeinen 650 bis 1050°C, vorzugsweise 820 bis 950°C und insbesondere 850 bis 920°C. Der beim Verfahren herrschende Druck ist nicht kritisch. Die Dehydrierung des Methanols kann bei Unterdruck, Normaldruck oder Überdruck, vorzugsweise aber bei einem Druck von ca. 1,2 bar durchgeführt werden.

Der Reaktor kann beispielsweise rohrförmig sein und besteht vorzugsweise aus Korund.

Das Methanol, das im Reaktor in gasförmigem Zustand vorliegt, kann als solches oder mit Kohlendioxyd gegebenenfalls auch mit einem inerten Gas, vorzugsweise Stickstoff, gemischt zur zur Umsetzung gebracht werden. Darüberhinaus kann auch Wasser dem Reaktionsgemisch zugegeben werden. Die zugesetzten Verbindungen wirken sich günstig auf die Langzeitaktivität des Katalysators aus, da diese Substanzen der Desaktivierung des Katalysators entgegenwirken. Im allgemeinen werden bis 1 Gew.-% Wasser und/oder bis 3 Mol-% Kohlendioxid, jeweils bezogen auf die zudosierte Methanolmenge, eingesetzt.

Das erfindungsgemäße Verfahren kann diskontinuierlich, vorzugsweise jedoch kontinuierlich durchgeführt werden. Dabei werden 0,1 bis 100 kg, vorzugsweise 0,5 bis 10 kg, Methanol pro Stunde und pro Kilogramm Katalysator, umgesetzt.

Die in den Beispielen angegebenen Meßgrößen wurden wie folgt berechnet:

$$\text{Umsatz (in \%)} = \frac{\text{umgesetztes Methanol (mol)}}{\text{zudosiertes Methanol (mol)}} \cdot 100$$

$$\text{Ausbeute (in \%)} = \frac{\text{gebildeter Formaldehyd (mol)}}{\text{zudosiertes Methanol (mol)}} \cdot$$

$$\text{Selektivität (in \%)} = \frac{\text{gebildeter Formaldehyd(mol)}}{\text{umgesetztes Methanol(mol)}} \cdot 100$$

Als Nebenreaktion trat eine geringe Bildung von Kohlenmonoxyd auf. Bei der Verfahrensdurchführung, bei der Wasser zum Reaktionsgemisch zugegeben wurde, wurden im Produktgas geringe Mengen an Kohlendioxyd und Methan festgestellt. In keinem Fall konnte Wasser, auch nicht von dem dem Reaktionsgemisch zudosierten, im Produktgas nachgewiesen werden.

**Beispiele**

Die Beispiele beschreiben jeweils die Dehydrierung von Methanol gemäß Gleichung (IIa).

1) Natriumcarbonat und Aluminiumhydroxyacetat wurden so vermischt, daß auf ein Mol Aluminium ein Mol Natrium kam. Das Gemenge wurde bei 900°C an Luft 3 Tage calciniert. Das so erhaltene Produkt wurde verrieben und zu Pellets mit einem Durchmesser von 6 mm verpreßt. Diese wurden zerkleinert und daraus eine Kornfraktion von 2 mm ± 1 mm Durchmesser ausgewählt. 1,71 g des so erhaltenen Katalysators mit einem Schüttvolumen von 2,2 ml wurden in ein Rohr aus $Al_2O_3$ mit 12 mm Innendurchmesser gegeben. Das zudosierte Methanol enthielt 0,1 Gewichtsprozent Wasser. Bei einer Reaktionstemperatur von 900°C und einem Zulauf von 3,97 mol/h (10 Mol-% Methanol, Rest Stickstoff ($N_2$)) betrug der Umsatz an Methanol 100 % und die Ausbeute an Formaldehyd 69,4 %.

2) Lithiumcarbonat und Aluminiumnitrat wurden so vermischt, daß auf ein Mol Lithium ein Mol Aluminium entfiel. Das Gemenge wurde 1 ½ Tage an Luft calciniert. Der so erhaltene Katalysator wurde zu Pellets mit 6 mm Durchmesser gepreßt. Diese wurden zerkleinert und daraus eine Kornfraktion mit 1 mm ± 0,5 mm Durchmesser ausgewählt. In ein $Al_2O_3$-Rohr mit 12 mm Innendurchmesser wurden 0,75 g dieser Substanz mit einem Schüttvolumen von 1 ml gegeben. Das zudosierte Methanol enthielt 0,05 Gewichtsprozent Wasser. Bei einer Reaktionstemperatur von 850°C und einem Zulauf von 3,97 mol/h (10 Mol-% Methanol, Rest $N_2$) betrug der Umsatz an Methanol 49,2% und die Ausbeute an Formaldehyd 23,9 %.

3) (Vergleich) 1,08 g $Al_2O_3$ (0,8 ml Schüttvolumen) mit 99,7 %iger Reinheit wurden in ein Quarzrohr mit 12 mm Innendurchmesser gegeben. Bei einer Reaktionstemperatur von 750°C und einem Zulauf von 1,33 mol/h (10 Mol-% Methanol in $N_2$) wurden 6,4 % des Methanols bei 100 %iger Selektivität zu Formaldehyd umgesetzt.

4) Aluminiumnitrat wurde mit Kaliumoxalat so vermischt, daß auf ein Mol Kalium ein Mol Aluminium kam. Das Gemisch wurde bei 850°C 5 Tage an Luft calciniert. Das so erhaltene Produkt wurde zu Pellets mit 6 mm Durchmesser gepreßt. Diese wurden zerkleinert und daraus eine Kornfraktion mit 3 mm ± 1 mm Durchmesser ausgewählt. 1,14 g des so erhaltenen Katalysators mit einem Schüttvolumen von 1,5 ml wurden in ein $Al_2O_3$-Rohr mit 12 mm Innendurchmesser gegeben. Das aufgegebene Methanol enthielt 0,05 Gewichtsprozent Wasser. Bei einer Reaktionstemperatur von 850°C und einem Zulauf von 5,53 mol/h (7,78 Mol-% Methanol in $N_2$) wurden 92,7 % des Methanols umgesetzt und eine Ausbeute von Formaldehyd von 40 % erzielt.

5) 1,14 g des nach Beispiel 4 produzierten Katalysators mit einem Schüttvolumen von 1,5 ml wurden in ein $Al_2O_3$-Rohr mit 12 mm Innendurchmesser gegeben. Das aufgegebene Methanol enthielt 0,05 Gewichtsprozent Wasser. Bei einer Reaktionstemperatur von 750°C und einem Zulauf von 2,76 mol/h (14,4 Mol-% Methanol, Rest $N_2$) wurden 71,7 % des zudosierten Methanols umgesetzt. Die Ausbeute an Formaldhyd betrug 14,9%.

6) Lithiumcarbonat, Natriumcarbonat und Aluminiumhydroxyacetat wurden so vermischt, daß auf ein Mol Lithium ein Mol Natrium und 2 Mole Aluminium kamen. Das Gemenge wurde an Luft 2 Tage bei 850°C getempert. Das Produkt wird zu Pellets von 6 mm Durchmesser verpreßt. Diese wurden zerkleinert und daraus eine Kornfraktion mit 1 mm ± 0,5 mm Durchmesser ausgewählt. 2,41 g von dieser Fraktion mit einem Schüttvolumen von 2,9 ml wurden in ein $Al_2O_3$-Rohr mit 12 mm Innendurchmesser gegeben. Das zudosierte Methanol enthielt 0,05 Gewichtsprozent Wasser. Bei einer Reaktionstemperatur von 900°C und einem Zulauf von 3,97 mol/h (10 Mol-% Methanol, Rest $N_2$) wurden 97,4% des Methanols umgesetzt und Formaldehyd in 72,8 %iger Ausbeute erhalten. Auch nachdem pro g Katalysator 120 g Methanol umgesetzt worden waren, war keine Aktivitätsabnahme feststellbar.

7) 0,93 g des nach Beispiel 6 hergestellten Katalysators mit 1,1 ml Schüttvolumen wurden in ein $Al_2O_3$-Rohr mit 12 mm Innendurchmesser gegeben. Bei einer Reaktionstemperatur von 750°C und einem Zulauf von 4,01 mol/h (10 Mol-% Methanol, 1 Molprozent $CO_2$, Rest $N_2$) wurden 22,4 % des Methanols zu Formaldehyd bei einer Selektivität von 100 % umgesetzt.

8) Beispiel 7 wurde wiederholt, jedoch enthielt das zudosierte Methanol 0,05 Gewichtsprozent Wasser. Bei einer Reaktionstemperatur von 900°C und einem Zulauf von 3,97 mol/h (10 Mol-% Methanol, Rest $N_2$) wurden 97,3 % des zudosierten Methanols umgesetzt. Die Selektivität betrug 69,4 %.

9) (Vergleichsbeispiel) Die Verfahrensweise des Beispiels 1 der DE-A-37 19 055 wurde herangezogen, jedoch wurde der Durchsatz an Methanol dem Beispiel 1 der vorliegenden Anmeldung angepaßt. Natriumcarbonat wurde stufenweise im Wasserstoffstrom kalziniert. Die Aufheizgeschwindigkeit betrug 5°C/min. Bei 100°C, 500°C und 600°C wurde die Temperatur jeweils drei Stunden belassen. Die Endtemperatur von 700°C wurde fünf Stunden gehalten. Von dem so erhaltenen Katalysator wurden 2 g in ein Quarzrohr von 10 mm Innendurchmeser gefüllt. Stündlich wurden 0,397 mol eines Stickstoff-Methanol-Gemisches mit 10 % Methanolgehalt durch die Katalysatorschüttung geleitet. Bei 700°C wurde das Methanol zu 22 % umgesetzt. Formaldehyd wurde mit 68 % Selektivität erhalten. Über den gesamten Zeitabschnitt wurden pro Gramm Katalysator etwa 1,5 g Formaldehyd gebildet, bis der Katalysator völlig verkokt (desaktiviert) war.

Der Versuch wurde bei 600°C wiederholt, wobei das Methanol zu 18 % umgesetzt und Formaldehyd mit 89 % Selektivität erhalten wurde.

## Patentansprüche

1. Verfahren zur Herstellung von Formaldehyd durch Dehydrierung von Methanol in Gegenwart eines Katalysators bei erhöhter Temperatur, dadurch gekennzeichnet, daß die Umsetzung bei einer Temperatur von 650 bis 1050°C unter Sauerstoffausschluß in Gegenwart von Aluminiumoxid, Alkalialuminat und/oder Erdalkalialuminat als Katalysator in einem Reaktor, dessen Innenwand ganz oder zum Teil aus Aluminiumoxid besteht, durchgeführt wird.

2. Verfahren nach Anspruch 1, dadurch gekennzeichnet, daß als Ausgangsmaterial Methanol allein oder eine Mischung von Methanol mit einem inerten Gas, vorzugsweise Stickstoff, eingesetzt wird.

3. Verfahren nach Anspruch 1 oder 2, dadurch gekennzeichnet, daß dem Ausgangsmaterial Wasser und/oder Kohlendioxid zudosiert wird.

4. Verfahren nach Anspruch 3, dadurch gekennzeichnet, daß bis zu 1 Gew.-% Wasser und/oder bis 3 Mol-% Kohlendioxid, bezogen auf die eingesetzte Methanolmenge, zudosiert wird.

5. Verfahren nach einem oder mehreren der Ansprüche 1 bis 4, dadurch gekennzeichnet, daß die Umsetzung bei einer Temperatur von 820 bis 950°C, insbesondere von 850 bis 920°C, durchgeführt wird.

6. Verfahren nach einem oder mehreren der Ansprüche 1 bis 5, dadurch gekennzeichnet, daß als Katalysator ein Alkalialuminat verwendet wird, das als Alkalimetall Lithium und/oder Natrium enthält.

7. Verfahren nach einem oder mehreren der Ansprüche 1 bis 6, dadurch gekennzeichnet, daß als Reaktor ein Rohr aus Korund verwendet wird.

8. Verfahren nach einem oder mehreren der Ansprüche 1 bis 7, dadurch gekennzeichnet, daß der Katalysator in Form von annähernd kugelförmigen Partikeln mit Durchmessern im Bereich von 1 bis 3 mm verwendet wird.

## Claims

1. A process for the preparation of formaldehyde by dehydrogenating methanol in the presence of a catalyst at elevated temperature, which comprises carrying out the reaction at a temperature of 650 to 1050°C with the exclusion of oxygen in the presence of aluminum oxide, alkali metal aluminate and/or alkaline earth metal aluminate as catalyst in a reactor whose inner wall is composed entirely or partly of aluminum oxide.

2. The process as claimed in claim 1, wherein the starting material used is methanol alone or a mixture of methanol with an inert gas, preferably nitrogen.

3. The process as claimed in claim 1 or 2, wherein water and/or carbon dioxide is metered into the starting material.

4. The process as claimed in claim 3, wherein up to 1% by weight of water and/or up to 3 mol% of carbon dioxide, relative to the amount of methanol used, is metered in.

5. The process as claimed in one or more of claims 1 to 4, wherein the reaction is carried out at a temperature of 820 to 950°C, in particular of 850 to 920°C.

6. The process as claimed in one or more of claims 1 to 5, wherein the catalyst used is an alkali metal aluminate which contains lithium and/or sodium as the alkali metal.

7. The process as claimed in one or more of claims 1 to 6, wherein the reactor used is a tube of corundum.

**8.** The process as claimed in one or more of claims 1 to 7, wherein the catalyst used is in the form of approximately spherical particles having diameters in the range of from 1 to 3 mm.

**Revendications**

**1.** Procédé de préparation de formaldéhyde par déshydrogénation de méthanol en présence d'un catalyseur à chaud, procédé caractérisé en ce que l'on effectue la réaction à une température de 650 à 1050°C à l'abri de l'oxygène en présence d'oxyde d'aluminium, d'un aluminate de métal alcalin et/ou d'un aluminate de métal alcalino terreux comme catalyseur, dans un réacteur dont la paroi interne est formée totalement ou partiellement d'oxyde d'aluminium.

**2.** Procédé selon la revendication 1, caractérisé en ce que l'on part de méthanol seul ou d'un mélange de méthanol et d'un gaz inerte, de préférence l'azote.

**3.** Procédé selon la revendication 1 ou 2, caractérisé en ce que l'on ajoute progressivement à la matière dont on part de l'eau et/ou du dioxyde de carbone.

**4.** Procédé selon la revendication 3, caractérisé en ce que l'on ajoute jusqu'à 1 % en poids d'eau et/ou jusqu'à 3 mol % de dioxyde de carbone par rapport à la quantité de méthanol employée.

**5.** Procédé selon l'une ou plusieurs des revendications 1 à 4, caractérisé en ce que l'on effectue la réaction à une température de 820 à 950°C, en particulier de 850 à 920°C.

**6.** Procédé selon l'une ou plusieurs des reevendications 1 à 5, caractérisé en ce que l'on utilise comme catalyseur de l'aluminate de lithium et/ou de sodium.

**7.** Procédé selon l'une ou plusieurs des revendications 1 à 6, caractérisé en ce que le réacteur est un réacteur tubulaire en corindon.

**8.** Procédé selon l'une ou plusieurs des revendications 1 à 7, caractérisé en ce que le catalyseur est en particules ayant à peu près la forme de petites billes, de diamètres de 1 à 3 mm.